# EUROPEAN PATENT SPECIFICATION

(11) **EP 0 452 457 B1**
(45) Date of publication and mention of the grant of the patent: **20.08.1997**
(21) Application number: 90916560.7
(22) Date of filing: 18.10.1990
(51) Int. Cl.: A61K 48/00, A61K 9/127, C12N 15/88

(54) **METHOD OF IN VIVO DELIVERY OF FUNCTIONING FOREIGN GENES**
VERFAHREN ZUR IN VIVO-VERABREICHUNG VON FUNKTIONSFÄHIGEN FREMDEN GENEN
PROCEDE D'ADMINISTRATION IN VIVO DE GENES ETRANGERS FONCTIONNELS

(30) Priority: 03.11.1989 US 431552
(43) Date of publication of application: 23.10.1991
(60) Divisional application: 97107677.3
(73) Proprietor: VANDERBILT UNIVERSITY, Nashville, TN 37240 (US)
(72) Inventor: BRIGHAM, Kenneth, L., Nashville, TN 37215 (US)
(74) Representative: Jaenichen, Hans-Rainer, Dr.
(86) International application number: US9005993
(87) International publication number: WO9106309

(56) References cited:
- EP-A- 0 027 662
- WO-A-85/04880
- US-A- 4 579 821
- PROCEEDINGS OF THE NATIONAL ACADEMY OF SCIENCES OF USA, vol. 80, no. 4, February 1983, WASHINGTON US, pages 1068-1072; CLAUDE NICOLAU ET AL.
- PROCEEDINGS OF THE NATIONAL ACADEMY OF SCIENCES OF USA, vol. 84, no. 22, November 1987, Washington US, pages 7851-7855; CHEN-YEN WANG ET AL
- Proc. Natl. Acad. Sci., Volume 84, issued November 1987, pages 7413-7417; FELGNER ET AL.
- Recent progress in Hormone Research, Volume 39, issued 1983, pages 353-385; PAVLAKIS ET AL.
- Methods in Enzymology, volume 149, issued 1987, pages 157-176; NICOLAU ET AL.
- American Journal of the Medical Sciences, Vol. 298, Nr. 4, 1989, pp. 278-281; Brigham et al

## Description

The present invention relates to the use of a complex, comprising a plasmid containing and capable of functionally expressing a gene complexed to cationic liposomes capable of transfecting DNA into a cell in a target organ of a mammal, for the manufacture of a medicament for expressing said gene principally in a target organ distal to the site of administration of said medicament. The present invention does not contemplate that said gene codes for chloramphenicol acetyltransferase (CAT).

Functioning DNA can be introduced by a variety of techniques resulting in either transient expression of the genes of interest, referred to as transient transfection, or permanent transformation of the host cells resulting from incorporation of the foreign DNA into the host genome; Berger, S.L., and A.R. Kimmel, 1987, "Guide to molecular cloning techniques". Methods in Enzymology 152,692-694. Potter, H., L. Weir and P. Leder, 1984, "Enhancer-dependent expression of human K immunoglobulin genes introduced into mouse pre-B lymphocytes by electroporation", Proc. Natl. Acad. Sci. USA 81, 7161-7165. Plasmids complexed with cationic liposomes, referred to as liposome-DNA complexes, have been used for the transfection of cell cultures.

The use of liposomes for such transfection has been referred to as lipofection. Of the many techniques used for transfecting cells, lipofection has been found to offer advantages of simplicity and high transfection efficiency; Brigham, K.L., B. Meyrick, B. Christman, L.C. Berry, and G. King, 1989, "Expression of a prokaryotic gene in cultured lung endothelial cells after lipofection with a plasmid vector", Am. J. Resp. Cell. Mol. Biol., 1,95-100. Felgner, P.L., T.R. Gadek, M. Holm, R. Roman, H.W. Chan, M. Wenz, J.P. Northrop, G.M. Rignold, and M. Danielsen, 1987, "Lipofection: A highly efficient, lipid-mediated DNA-transfection procedure", Proc. Natl. Acad. Sci. USA 84, 7413-7417.

Kaneda et al, Science 243, January 1989, pages 375-378, discloses an in vivo transfection technique wherein plasmid-DNA and nuclear protein were cointroduced into nondividing cells in rat liver by injection into the portal veins of adult rats, the plasmid-DNA being carried into liver cell nuclei by the nuclear protein; Wu et al., Journal of Biological Chemistry 263, No. 29, issue of Oct. 15, pages 14621-14624, 1988 disclose an in vivo transfection technique wherein plasmids are bound to ligands for specific hepatic galactose receptor complexes injected IV and showed specific gene expression in the liver.

Brigham, K.L., Meyrick, B., Christman, B., Magnuson, M., King, G., and Berry, L.C.Jr., 1989, "Rapid Communication: In vivo transfection of murine lungs with a functioning prokaryotic gene using a liposome vehicle", The American Journal of the Medical Sciences 298, 278-281, describes intravenous and intratracheal administration of a prokaryotic gene encoding chloramphenicol acetyltransferase (CAT) into mice. However, the authors state: "It is not entirely clear why expression of the CAT gene in our studies appeared to be predominantly in the lungs when the liposome-DNA complex was given intravenously."

The present invention provides an in vivo lipofection technique resulting in expression of desired foreign products in cells of a mammalian organ.

In accordance with the present invention, there is provided a use allowing the principal expression of a foreign gene in cells of a mammalian organ in vivo. The liposomes used are complexed to a gene expression inducer of desired gene products and injected into a mammal. The inducer is complexed to a cationic liposome carrier. The inducer is lipofected into cells of the mammalian organ. Gene expression and cellular function of the inducer is activated to produce the desired gene products in the cells of the organ.

Thus, the invention relates to the subject matter as defined in the claims.

Other advantages of the present invention will be readily appreciated as the same becomes better understood by reference to the following detailed description when considered in connection with the accompanying drawings therein:
Figure 1 shows expression of a human growth hormone gene in the lungs of mice following intravenous injection of a plasmid containing the coding region for human growth hormone driven by a metallothionein promoter. Data are expressed as ng hGH per 24 hours per gm tissue and all points are means of data from 2 different animals. Control values were subtracted from the data in the figure. There was minimal expression of the foreign gene in either kidneys or liver, but the gene was expressed in the lungs with a time course similar to that seen in cultured endothelial cells.
Figure 2 shows CAT activity in the organs of mice 72 hours following injection of the DNA-liposome complex.
Figure 3 shows lung CAT activity for the 3 days following injection of the liposome-DNA complex.

Generally, the present invention provides a use allowing principal expression of a foreign gene in cells of a mammalian organ in vivo. A gene expression inducer of desired gene products is injected into a mammal. For example, the gene expression inducer could be genetic material, such as DNA or RNA. The gene products could be, for example, a polypeptide or protein such as a growth hormone.

The inducer is complexed to a cationic liposome carrier. An example of such a carrier is LIPOFECTIN-, manufactured by Bethesda Research Laboratories Life Technologies, Inc. of Gaithersburg, MD. LIPOFECTIN- reagent has previously been used for transfecting cells in vitro. LIPOFECTIN- reagent contains the cationic lipid LN-[1-(2,3-Dioleyloxy) propyl]-N,N,N-trimethylammonium chloride which complexes with the DNA by ionic interaction. Generally, the complex then fuses with cell membrane and is transfected into the cell. Methods of utilizing lipofection as highly efficient transfection procedure have been reported by Felgner et al., Proc. Natl. Acad. Sci. U.S.A. 84, pages 7413-7417, Nov. 1987; and by Felgner et al., Nature 337, 26 Jan. 1989, pages 387-388.

Utilizing the cationic liposome carrier, the genetic material is lipofected into the cells of the mammalian organ. Gene expression and cellular function of the genetic material is activated to produce the desired foreign products in the cells of the organ. That is, expression of particular expression vectors of the genetic material can be promoted.

More specifically, plasmid bound to the carrier, the plasmid containing the predetermined genetic material, is intravenously injected into the blood stream of the mammal in proximity to the organ for transfection. It has been found that when such plasmid carrier complexes are injected intratracheally, a high degree of transfection occurs in the cells of the lung. The genetic expression is transient. The transient expression in vivo of the transfected gene provides utility for gene therapy, including prevention and treatment of acute or subacute conditions which are not necessarily related to any genetic abnormality. The acute abnormality could be irradicated by the transient treatment, prolonged treatment not being necessary. Hence, the present invention could be used not only as a tool for correcting genetic abnormalities, but also as a new category of therapy which could broadly be applicable to human disease states. Accordingly, there is an advantage to the transiency of the plasmid transfection.

The examples illustrate the invention.

### EXPERIMENTAL DATA

### 1. Expression of Coding Region for Human Growth Hormone.

### Methods

### Plasmid Descriptions

Plasmid constructs obtained from Nichols Institute Diagnostics, San Juan Capistrano, California, were used. The plasmid, pXGH5, was a 6.7 kilobase construct in pUC12 containing the coding region for human growth hormone driven by a mouse metallothionein (mMT-1) promoter. Plasmid DNA was propagated in Escherichia coli. Plasmid DNA was isolated by alkaline lysis and purified by isopyknic equilibration gradient centrifugation in cesium chloride and ethidium bromide; Maniatis, T., E.F. Fritsch, and J. Sambrook, 1982, "Molecular Cloning, A Laboratory Manual", Cold Spring Harbor, Cold Spring Harbor Laboratory.

### In vivo Transfection

In vivo studies were carried out in 6 week old specific pathogen free International Cancer Research/Harlan Sprague-Dawley female mice (body weight 20-25 gm). Beginning 24 hours prior to DNA injection and continuing to the end of the experiment, all mice were given 5000 ppm ZnSO₄ in their drinking water. This amount of zinc has been shown to activate the metallothionein promoter in transgenic mice; Palmiter, R.D., R.L. Brinster, R.E. Hammer, M.E. Trumbauer, M.G. Rosenfeld, N.C. Birnberg, and R.M. Evans, 1982, "Dramatic growth of mice that develop from eggs microinjected with metallothionein-growth hormone fusion genes"; Nature 300,611-615. Each mouse was injected intravenously through a tail vein puncture with a 25 gauge needle with 30 ug pXGH5 DNA complexed to 150 ug liposome (Lipofectin TM) in a total volume of 300 ul. Mice were killed 1, 3 and 5 days following DNA injection by an intraperitoneal injection of pentobarbital and the lungs, liver and kidneys were removed under sterile conditions. The organs were weighed, minced and placed in 60 mm Petri dishes to which were added 2ml medium 199. The dishes were incubated for 24 hours at 37°C in 5% CO₂ after which the contents were centrifuged and growth hormone assays were performed on the medium. Growth hormone production was calculated as the product of hGH concentration and the total volume of medium (2ml), normalized to organ weight and expressed as ng hGH per gm tissue per 24 h. Identical measurements were made in 5 non-transfected control mice.

### 2. Results

### In vivo Transfection

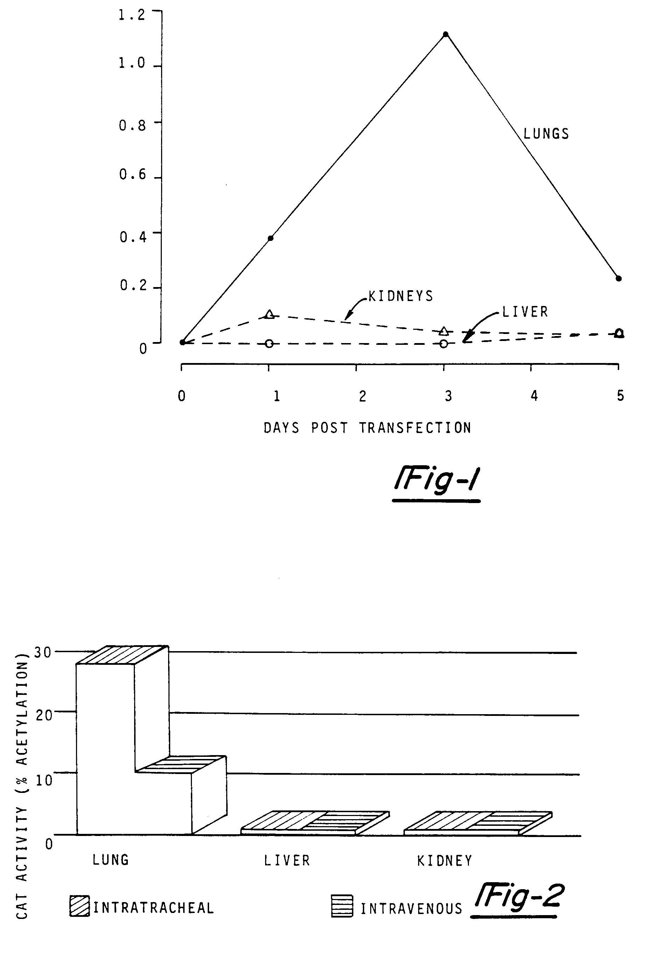

Medium from 24 hour incubations of lungs, liver and kidneys removed from mice which were not injected with the plasmid-liposome complex showed very low levels of immunoreactive hGH. Calculated hGH production in ng per gm tissue per 24 hours was: lungs = 0.26; kidneys = 0.26; liver = 0.06 (mean of N = 5 in all cases). Expression of the hGH gene in mouse organs removed at different intervals following intravenous injection of plasmid-liposome complex is shown in Figure 1. There was little production of growth hormone by either kidneys or liver. However, lungs removed from the transfected mice produced substantial amounts of growth hormone. hGH production was increased by 24 hours after injection, peaked at 3 days after injection and by 5 days was declining. This time course is similar to that seen in cultured endothelial cells.

### 3. Experiment 2

A plasmid which contained a prokaryotic (bacterial) gene, chloramphenicol acetyltransferase (CAT), was bound to LIPOFECTIN- reagent. The liposome-DNA complex was injected into mice. The lungs of the mice were shown to express CAT gene. CAT is not present in any mammalian cells normally and measurement of CAT activity is thus an absolute index of successful expression of the gene. Previously, this plasmid construct was commonly used in cultured cells where CAT activity is proportional to mRNA levels.

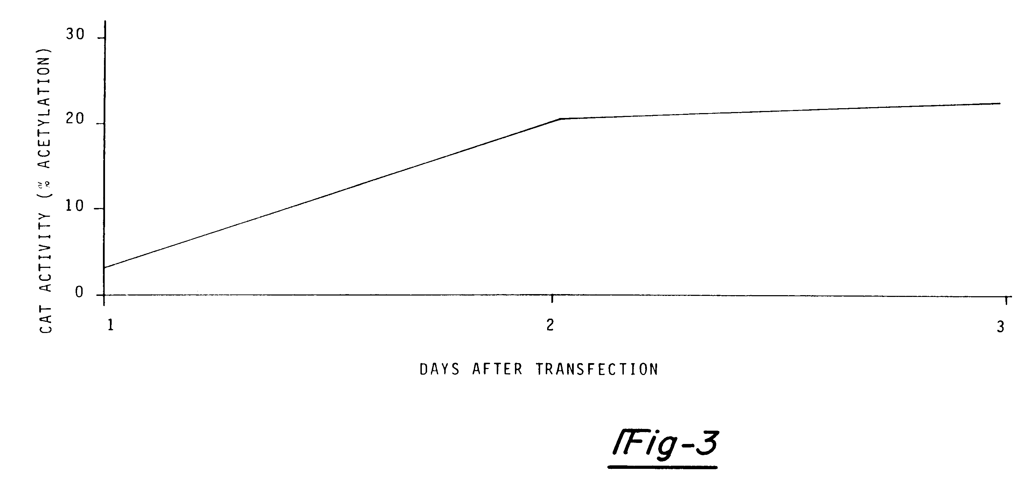

Figure 2 shows CAT activity in the organs of mice 72 hours following injection of the DNA-liposome complex and Figure 3 shows lung CAT activity for the three days following intravenous injection of the lipsome-DNA complex. There is a large amount of activity in the lungs with no detectable activity in peripheral organs. It is expected that the specific transfection of the lungs is probably because of the lungs being the first organ distal to the site of injection. When the DNA-liposome complex is given intratracheally, as indicated in Figure 2, larger amounts of activity appear in the lungs than with intravenous studies. Intraperitoneal injection show no CAT activity in any organ up to 6 days following injection.

### 4. Discussion

The present invention permits introduction of foreign genes directly into host cells by injection of the genetic material. DNA bound to specifically synthesized cationic liposomes can introduce plasmids and other gene vectors into cultured cells with high efficiency. It is suspected that the plasmid does not enter the host genome and does not replicate in a mammalian cell so that the gene expression is transient, in the present examples lasting for greater than a week.

Following intravenous injection of the DNA-liposome complex, the major expression of the gene was found in the lungs. This is interpreted to mean that the principal organ transfected following intravenous injection is the first capillary bed downstream from the injection site. Accordingly, it should be possible to selectively transfect organs by injecting the liposome DNA complex in the artery supplying the organ.

Previously, Iannuzzi and Associates transfected human airway epithelial cells with a plasmid containing the prokaryotic gene, CAT, driven by an SV40 promoter by electroporation, Iannuzzi, M.C. , J.L. Weber, J. Yankaskas, R. Boucher, and F.S. Collins, 1988, "The introduction of biologically active foreign genes into human respiratory epithelial cells using electroporation", AM. Rev. Resp. Dis. 138,965-968, These experiments showed expression of the CAT gene in transfected cells, Zwiebel and co-workers transformed rabbit aorta endothelial cells in culture by infecting them with a retroviral vector containing either hGH or adenosine deaminase coding regions driven by an SV40 promoter, Zwiebel, J.A., S.M. Freeman, P.W. Kantoff, K. Cornetta, U.S. Ryan, and W.F. Anderson, 1989, "High-level recombinant gene expression in rabbit endothelial cells transduced by retroviral vectors", Science 243,220-242. The experiment showed expression of the genes in the transformed cells which persisted through many generations in culture. The inventors of the present invention earlier showed that bovine lung endothelial cells could be transfected by lipofection at very high efficiency with a plasmid containing the CAT coding region driven by a Rous sarcoma virus promoter, Brigham, K.L., B. Meyrick, B. Christman, L.C. Berry, and G. King, 1989, "Expression of a prokaryotic gene in cultured lung endothelial cells after lipofection with a plasmid vector", Am. J. Resp. Cell Mol. Biol. 1,95-100.

The studies detailed above demonstrate the use of transfecting the lung of intact animals with a functioning gene encoding a physiologically relevant secreted human protein driven by the metallothionein promoter. The time course of expression of the transfected gene in the lung was similar to the time course of expression over the same gene in cultured lung endothelial cells (experiments not reported).

Gene therapy has generally been conceived as principally applicable to genetic deficiency diseases where permanent cure may be effected by introducing a functioning gene, Friedmann , T., 1989, "Progress toward human gene therapy". Science 244,1275-1281. However, a much larger group of diseases might be treatable by transiently engineering host cells to produce beneficial proteins. For example, the intracellular enzymes, superoxide dismutase and catalase, may be crucial protectors of the lungs from oxidant injury and increased intracellular levels of these proteins might protect the lung from injury of a variety of causes. Transient increase in endothelial prostaglandin synthetase resulting in increased production of prostacyclin and prostaglandin E₂ might be beneficial in several diseases. The secreted antiprotease, alpha-1 antitrypsin, may be important in the pathogenesis of emphysema and other lung diseases and genetic engineering of lung cells to produce this protein could be therapeutic in human disease states, Garver, R.I., J.F. Mornex, T. Nukiwa, M. Brantly, M. Courtney, J.P. LeCocq and R.G. Crystal, 1986, "Alpha-1 antitrypsin genes", N. Engl. J. Med. 314,762-766. Numerous other theoretical possibilities could be suggested.

The present data demonstrate that lung cells in living animals can be engineered by transfection to express foreign genes encoding both intracellular and secreted proteins. Several promoters, including those which permit experimental control of gene expression, can be used to drive expression of the transfected gene. Use of plasmid vectors helps to assure transient expression of the gene. This approach will permit elucidation of molecular mechanisms effecting gene expression in specific lung cells and specific investigation of the rolls of various proteins in lung cell responses. In addition, in vivo transient transfection may make gene therapy applicable to a broad range of human diseases.

## Claims

1. Use of a complex, comprising a plasmid containing and capable of functionally expressing a gene complexed to cationic liposomes capable of transfecting DNA into a cell in a target organ of a mammal, for the manufacture of a medicament for expressing said gene principally in a target organ distal to the site of administration of said medicament, wherein said principal expression in said target organ is caused by selection of said site to be proximal to the target organ, with the provision that said gene does not code for chloramphenicol acetyl transferase (CAT).

2. The use of claim 1 in which complexation is based on ionic interaction.

3. The use of claim 1 or 2, wherein the target organ is the first capillary bed downstream from the injection site.

4. The use of claim 1 or 2, wherein the medicament is for administration into the artery supplying the target organ.

5. The use of any one of claims 1 to 3, wherein the target organ is the lungs.

6. The use of claim 1 or 2, wherein the target organ is the lungs and the medicament is for administration by intratracheal injection.

7. The use of any one of claims 1 to 6, wherein the medicament further comprises an activator capable of activating transient expression of the gene.

8. The use of claim 7, wherein the activator is zinc.

9. The use of claim 7 or 8, wherein the activator is for administration by ingestion.

10. The use of any one of claims 1 to 9, wherein the coding region codes for human growth hormone.

11. The use of any one of claims 1 to 10, wherein the cationic liposomes have the transfection characteristics of LN-[1-(2,3-dioleyloxy)propyl]-N,N,N-trimethylammonium chloride.

## Patentansprüche

1. Verwendung eines Komplexes enthaltend ein Plasmid, das ein Gen enthält und in der Lage ist, dieses funktionell zu exprimieren, komplexiert an kationische Liposomen, die in der Lage sind, DNA in eine Zelle in einem Zielorgan eines Säugers zu transfizieren, zur Herstellung eines Arzneimittels zur Expression des Genes hauptsächlich in einem Zielorgan distal von der Stelle der Verabreichung des Arzneimittels, wobei die hauptsächliche Expression in dem Zielorgan hervorgerufen wird durch die Wahl der Stelle, so daß sie proximal zum Zielorgan liegt, unter der Voraussetzung, daß das Gen keine Chloramphenicolacetyl-Transferase (CAT) codiert.

2. Verwendung nach Anspruch 1, wobei die Komplexierung auf ionischen Wechselwirkungen beruht.

3. Verwendung nach Anspruch 1 oder 2, wobei das Zielorgan das erste Kapillarbett stromabwärts der Injektionsstelle ist.

4. Verwendung nach Anspruch 1 oder 2, wobei das Arzneimittel zur Verabreichung in die Arterie vorgesehen ist, die das Zielorgan versorgt.

5. Verwendung nach einem der Ansprüche 1 bis 3, wobei das Zielorgan die Lungen sind.

6. Verwendung nach Anspruch 1 oder 2, wobei das Zielorgan die Lungen sind und das Arzneimittel zur Verabreichung durch intratracheale Injektion vorgesehen ist.

7. Verwendung nach einem der Ansprüche 1 bis 6, wobei das Arzneimittel ferner einen Aktivator enthält, der in der Lage ist, eine transiente Expression des Gens zu aktivieren.

8. Verwendung nach Anspruch 7, wobei der Aktivator Zink ist.

9. Verwendung nach Anspruch 7 oder 8, wobei der Aktivator zur Verabreichung durch Nahrungsaufnahme vorgesehen ist.

10. Verwendung nach einem der Ansprüche 1 bis 9, wobei der codierende Bereich humanes Wachstumshormon codiert.

11. Verwendung nach einem der Ansprüche 1 bis 10, wobei die kationischen Liposomen die Transfektionseigenschaften von LN-[1-(2,3-Dioleyloxy)-propyl]-N,N,N-trimethylammoniumchlorid aufweisen.

## Revendications

1. Utilisation d'un complexe, comprenant un plasmide contenant et capable d'exprimer fonctionnellement un gène et complexé à des liposomes cationiques capables de transfecter de l'ADN dans une cellule d'un organe cible d'un mammifère, pour la fabrication d'un médicament en vue d'exprimer principalement ledit gène dans un organe cible distal par rapport au site d'administration dudit médicament, ladite expression principale dans ledit organe cible provenant du fait que ledit site est sélectionné pour être proximal par rapport à l'organe cible, étant prévu que ledit gène ne code pas pour la chloramphénicol acétyl transférase (CAT).

2. Utilisation selon la revendication 1, dans laquelle la complexation est basée sur une interaction ionique.

3. Utilisation selon la revendication 1 ou 2, dans laquelle l'organe cible est le premier lit de capillaires situé en aval du site d'injection.

4. Utilisation selon la revendication 1 ou 2, dans laquelle le médicament est destiné à l'administration dans l'artère alimentant l'organe cible.

5. Utilisation selon l'une quelconque des revendications 1 à 3, dans laquelle l'organe cible est le poumon.

6. Utilisation selon la revendication 1 ou 2, dans laquelle l'organe cible est le poumon et le médicament est destiné à l'administration par injection intra-trachéale.

7. Utilisation selon l'une quelconque des revendications 1 à 6, dans laquelle le médicament comprend en outre un activateur capable d'activer l'expression momentanée du gène.

8. Utilisation selon la revendication 7, dans laquelle l'activateur est le zinc.

9. Utilisation selon la revendication 7 ou 8, dans laquelle l'activateur est destiné à une administration par ingestion.

10. Utilisation selon l'une quelconque des revendications 1 à 9, dans laquelle la région codante code pour l'hormone de croissance humaine.

11. Utilisation selon l'une quelconque des revendications 1 à 10, dans laquelle les liposomes cationiques ont les caractéristiques de transfection du chlorure de LN-[1-(2,3-dioléyloxy)-propyl]-N,N,N-triméthyl ammonium.
